# EUROPEAN PATENT SPECIFICATION

(11) **EP 4 011 216 B1**
(45) Date of publication and mention of the grant of the patent: **20.05.2026**
(21) Application number: 20850115.5
(22) Date of filing: 30.07.2020
(51) Int. Cl.: A23L 33/135, A23L 2/52, C12N 1/20

(54) **BIFIDOBACTERIA HAVING LOW ACTIVITY OF INDUCING INFLAMMATORY CYTOKINE PRODUCTION BUT HIGH ACTIVITY OF INDUCING ANTI-INFLAMMATORY CYTOKINE PRODUCTION**
BIFIDOBACTERIUM MIT GERINGER AKTIVITÄT ZUR INDUZIERUNG DER ERZEUGUNG VON ENTZÜNDLICHEN CYTOKINEN UND HOHER AKTIVITÄT ZUR INDUZIERUNG DER ERZEUGUNG VON ENTZÜNDUNGSHEMMENDEN CYTOKINEN
BIFIDOBACTERIUM AYANT UNE FAIBLE ACTIVITÉ D'INDUCTION DE LA GÉNÉRATION DE CYTOKINES INFLAMMATOIRES ET UNE ACTIVITÉ ÉLEVÉE D'INDUCTION DE LA GÉNÉRATION DE CYTOKINES ANTI-INFLAMMATOIRES

(30) Priority: 07.08.2019 JP 2019145173
(43) Date of publication of application: 15.06.2022
(73) Proprietor: NISSIN FOODS HOLDINGS CO., LTD., Yodogawa-ku Osaka-shi, Osaka 532-8524 (JP)
(72) Inventor: SUNADA, Yosuke, Osaka-shi Osaka 532-8524 (JP); IGI, Akemi, Osaka-shi Osaka 532-8524 (JP); UEHARA, Kazuya, Osaka-shi Osaka 532-8524 (JP); TANII, Yusuke, Osaka-shi Osaka 532-8524 (JP)
(74) Representative: Mewburn Ellis LLP
(86) International application number: PCT/JP2020/029299
(87) International publication number: WO 2021/024915

(56) References cited:
- JP-A- 2006 521 537
- JP-A- 2012 526 528
- JP-A- 2013 509 204
- US-A1- 2012 064 118
- US-A1- 2012 064 118
- US-A1- 2012 076 829
- US-A1- 2013 224 167
- US-A1- 2013 224 253
- US-A1- 2017 252 382
- US-A1- 2017 252 382
- TAKAHASHI HIDENORI ET AL: "Comparative Analysis of the Properties of Bifidobacterial Isolates From Fecal Samples of Mother-Infant Pairs", JOURNAL OF PEDIATRIC GASTROENTEROLOGY AND NUTRITION, vol. 51, no. 5, 1 November 2010 (2010-11-01), US, pages 653 - 660, XP093335682, ISSN: 0277-2116, DOI: 10.1097/MPG.0b013e3181f0e032
- GAD MONIKA ET AL: "Regulation of the IL-10/IL-12 axis in human dendritic cells with probiotic bacteria", FEMS IMMUNOLOGY & MEDICAL MICROBIOLOGY, vol. 63, no. 1, 1 October 2011 (2011-10-01), pages 93 - 107, XP055791378, ISSN: 0928-8244, DOI: 10.1111/j.1574-695X.2011.00835.x
- GAD MONIKA, RAVN PETER, SØBORG DITTE A., LUND-JENSEN KARINA, OUWEHAND ARTHUR C., JENSEN SIMON S.: "Regulation of the IL -10/ IL -12 axis in human dendritic cells with probiotic bacteria", FEMS IMMUNOL MED MICROBIOL, vol. 63, 2011, pages 93 - 107, XP055791378

## Description

### TECHNICAL FIELD

The present invention relates to a strain of infant gut-derived bifidobacteria having a low activity of inducing inflammatory cytokine production but a high activity of inducing anti-inflammatory cytokine production, and a beverage or food containing the bacterial cells thereof. The present invention relates to *Bifidobacterium breve.*

### BACKGROUND ART

Bifidobacteria have been used for various purposes such as impartation of taste or flavor, enhancement of nutrition, and improvement of food storability in many processed food or drink products such as fermented dairy products such as yogurt and various pickles. In recent years, bifidobacteria have been attracting attention for their effective bioactivities, such as anti-allergic and immunostimulating effects, and vigorous research has been conducted. The isolation of *B. breve* strains from feces of Japanese infants is described in Takahashi Hidenori et al., Comparative Analysis of the Properties of Bifidobacterial Isolates From Fecal Samples of Mother-Infant Pairs, in: Journal of Pediatric Gastroenterology and Nutrition, vol. 51, no. 5, November 2010, pages 653-660.

The immune system is controlled by immunomodulators called cytokines. Here, as an example of cytokines, interleukin (IL) is known. For example, IL-10 is an anti-inflammatory cytokine produced by immune cells such as dendritic cells and regulatory T cells. IL-10 has the capability of suppressing an immune response, and plays a role in suppressing an excessive immune response which causes autoimmune diseases, inflammatory diseases, allergic diseases, and the like.

On the other hand, IL-12 is an inflammatory cytokine produced by immune cells such as dendritic cells and T helper 2 (Th2) cells. IL-12 has the capability of promoting inflammation.

Increasing IL-10, which is an anti-inflammatory cytokine, is considered to be effective in controlling the destruction of connective tissue seen in chronic inflammatory diseases, and Patent Literature 1 discloses that a strain of the genus *Lactococcus* was obtained as a microorganism that efficiently inducing IL-10 production. In addition, from the viewpoint of suppressing inflammation, suppressing the production of IL-12, which is an inflammatory cytokine, is also considered to be important. Therefore, if a material which promotes IL-10 production and does not induce IL-12 production, that is, a material that increases the ratio of the amount of IL-10 production/amount of IL-12 production, can be searched, then a material having a high anti-inflammatory effect, or a product using the same can be provided, and the alleviation of autoimmune diseases, inflammatory diseases and allergic diseases can be expected.

### CITATION LIST

### PATENT LITERATURE

Patent Literature 1: Japanese Patent Laid-Open No. 2005-154387

### SUMMARY OF INVENTION

### TECHNICAL PROBLEM

The present invention aims to provide infant gut-derived bifidobacteria having a low activity of inducing inflammatory cytokine production but a high activity of inducing anti-inflammatory cytokine production, and food or drink products containing the bifidobacteria.

### SOLUTION TO PROBLEM

The present inventors have screened a large number of bifidobacteria using infant feces as the isolation source. As a result, the present inventors found that there are bifidobacteria having a low activity of inducing inflammatory cytokine production but a high activity of inducing anti-inflammatory cytokine production and completed the present invention.

That is, the first invention of the present application is bifidobacteria belonging to the genus *Bifidobacterium,* which has a low activity of inducing inflammatory cytokine production but a high activity of inducing anti-inflammatory cytokine production. wherein the bifidobacteria are *Bifidobacterium breve* strain N708 (NITE BP-02958).

The second invention of the present application is a food or drink product containing the bifidobacteria according to the first invention of the present application.

### ADVANTAGEOUS EFFECTS OF INVENTION

The bifidobacteria of the present invention have a low activity of inducing inflammatory cytokine production but a high activity of inducing anti-inflammatory cytokine production.

### BRIEF DESCRIPTION OF DRAWINGS

[Figure 1] Figure 1 is a diagram comparing the amount of IL-10 production (pg/ml) between the strain of the present invention and the comparative strains.
[Figure 2] Figure 2 is a diagram comparing the amount of IL-12 production (pg/ml) between the strain of the present invention and the comparative strains.
[Figure 3] Figure 3 is a diagram comparing the ratio of the amount of IL-10 production/amount of IL-12 production between the strain of the present invention and the comparative strains.

### Description of Embodiments

The following will describe in detail the present invention.

### 1. Bifidobacterium breve strain N708 (NITE BP-02958)

The bifidobacteria of the present invention are a *Bifidobacterium breve.* The denotation N708 in the present invention is a number uniquely assigned to the strain by NISSIN FOODS HOLDINGS CO., LTD. The strain of the present invention was first isolated from infant feces by the present inventors.

The *Bifidobacterium breve* strain N708 of the present invention was deposited under the following conditions.
(1) Depositary organization name: Patent Microorganisms Depositary Center, National Institute of Technology and Evaluation
(2) Contact: #122, 2-5-8 Kazusakamatari, Kisarazu-shi, Chiba 292-0818
(3) Accession number: NITE BP-02958
(4) Designation for identification: N708
(5) Date of deposit: May 29, 2019

The mycological properties of the *Bifidobacterium breve* strain N708 of the present invention are as shown in Tables 1 and 2 below. These mycological properties are based on the method described in Bergey's manual of systematic bacteriology Vol.2 (1986). Table 1 shows the form and the like of the present strain, and Table 2 shows the results of the tests on physiological and biochemical properties using Api 20A (manufactured by bioMérieux). In Table 2, "+" indicates positive and "-" indicates negative.

**[Table 1]**

| Culture Temperature (°C) | | 37°C | |
|---|---|---|---|
| Cell Morphology | | Bacillus | |
| | | 0.5×2.0-4.0 µm | |
| Gram Stain | | Positive | |
| Colony Morphology | Medium | | GAM Medium |
| | Culture time | | 24 hours |
| | Diameter | | 1.0 mm |
| | Color | White to Cream | |
| | Form | | Circular |
| | Elevation | | Raised |
| | Margin | | Entire |
| | Texture | | Smooth |
| | Transparency | | Translucent |
| | Viscosity | | Butter-like |
| Presence of Spore | | - | |
| Motility | | - | |
| Catalase Reaction | | - | |
| Growth Temperature Test | 37°C | + | |
| | 45°C | + | |

| | | | |
|---|---|---|---|
| +: Positive, -: Negative | | | |

**[Table 2]**

| | Reaction/Enzyme | Result | | Reaction/Enzyme | Result |
|---|---|---|---|---|---|
| 1 | Indole Production | - | 11 | Gelatin Hydrolysis | - |
| 2 | Urease | - | 12 | Esculin Hydrolysis | + |
| 3 | Glucose Fermentation | + | 13 | Glycerol Fermentation | - |
| 4 | Mannitol Fermentation | - | 14 | Cellobiose Fermentation | - |
| 5 | Lactose Fermentation | + | 15 | Mannose Fermentation | + |
| 6 | Saccharose Fermentation | + | 16 | Melezitose Fermentation | - |
| 7 | Maltose Fermentation | - | 17 | Raffinose Fermentation | + |
| 8 | Salicin Fermentation | - | 18 | Sorbitol Fermentation | - |
| 9 | Xylose Fermentation | - | 19 | Rhamnose Fermentation | - |
| 10 | Arabinose Fermentation | - | 20 | Trehalose Fermentation | - |

### 2. Evaluation Test on Ability to Induce IL-10 and IL-12 Production

The *Bifidobacterium breve* strain N708 of the present invention is a strain having a high ability to induce anti-inflammatory IL-10 production and a low ability to induce inflammatory IL-12 production, as shown in the examples described later. The evaluation of the ability to induce IL-10 and IL-12 production was conducted according to the following test method.

### <Preparation of Bacterial Cells>

For the preparation of the subject (killed bacterial cell powder) used in the evaluation test on the ability to induce IL-10 and IL-12 production, the bifidobacteria were pre-cultured and main-cultured in the GAM medium (Nissui) shown in Table 3 at 37°C for 24 hours. Next, the grown cells in the main culture solution were centrifuged and collected, and the separated cells were washed with sterile water three times, sterilized by heat, and then frozen. Then, the cells were freeze-dried using a freeze dryer to obtain a dried killed bacterial cell powder.

**[Table 3]**

| | |
|---|---|
| Peptone | 10 g |
| Soy Peptone | 3 g |
| Proteose Peptone | 10 g |
| Serum Digestive Powder | 13.5 g |
| Yeast Extract | 5.0 g |
| Meat Extract | 2.2 g |
| Liver Extract | 1.2 g |
| Glucose | 3.0 g |
| Potassium Dihydrogen Phosphate | 2.5 g |
| Sodium Chloride | 3.05 g |
| Soluble Starch | 5.0 g |
| L-Cysteine Hydrochloride | 0.3 g |
| Sodium Thioglycolate | 0.3 g |
| Distilled Water | 1000 ml |

### <Evaluation Test on Ability to Induce IL-10 and IL-12 Production>

In order to evaluate the ability to Induce IL-10 and IL-12 production of the strain of the present invention, human peripheral blood CD14+ monocytes were purchased from Lonza Japan Ltd. and were induced to differentiate into dendritic cells according to a reference literature (Monika et al., Regulation of the IL-10/IL-12 axis in human dendritic cells with probiotic bacteria. (2011) FEMS Immunol Med Microbiol. 63:93-107.). That is, human peripheral blood CD14+ monocytes were induced to differentiate into dendritic cells by culturing them in RPMI-1640 medium containing 20 ng/mL of GM-CS (Granulocyte Macrophage colony-stimulating Factor), 20 ng/mL of IL-4 and 10% FBS for 6 days under the conditions of 37°C and 5% CO₂. After the cells were collected, the cell count was adjusted, and 1 x 10⁵ cells/well were dispensed into a 96-well culture plate. The dried killed bacterial cell powder obtained above was added thereto to be 100 ng/mL, and then the mixture was cultured for 6 hours under the conditions of 37°C and 5% CO₂. Furthermore, an IL-12p70 inducer (0.1 µL/mL Lipopolysaccharide, 20 ng/mL IFN-y and 50 ng/mL TNF- α) was added, and the mixture was cultured for 18 hours. Note that the production of IL-12p70, which is an inflammatory cytokine, increases when the dendritic cells to which the IL-12p70 inducer was added are placed in a stressful environment. In the present invention, the IL-12p70 inducer was added to the dendritic cells for the purpose of searching for a strain that induces IL-10 production and does not induce IL-12 production, even when the dendritic cells are placed in a stressful environment. After the culture, the culture supernatant was collected, and IL-10 in the culture supernatant was measured by the IL-10 Human Uncoated ELISA Kit (Invitrogen), and IL-12 was measured by Human IL-12p70 Uncoated ELISA (Invitrogen).

### 3. Food or Drink Products

The bifidobacteria of the present invention can be used by being incorporated in a food or drink product. The bifidobacteria of the present invention can be suitably used particularly in beverages, and for example, fermented milk and lactic acid bacteria beverages can be considered. According to the current Ministerial Ordinance Concerning Compositional Standards etc. for Milk and Milk Products, 1.0 x 10⁷ cfu/mL or more is required as a compositional standard for fermented milks (solids not fat of 8.0% or more) and milk product lactic acid bacteria beverages (solids not fat of 3.0% or more), and 1.0 x 10⁶ cfu/mL or more for lactic acid bacteria beverages (solids not fat of less than 3.0%), and the above cell count can be achieved by growing the lactic acid bacteria in a fermented solution of milk or the like or by growing the lactic acid bacteria in the form of the final product. In addition to fermented milks and lactic acid bacteria beverages containing bifidobacteria, the bifidobacteria of the present invention can also be used in milk products such as butter, processed egg products such as mayonnaise, and sweet buns such as butter cake. Moreover, the bifidobacteria of the present invention can be suitably used in processed food products such as instant noodles and cookies. In addition to the above, the food of the present invention may be in a form (for example, a powder, a granule, a capsule, and a tablet) formulated by adding appropriate carriers and additives together with the bifidobacteria as needed.

It is also useful to incorporate the bifidobacteria of the present invention in foods for specified health use, dietary supplements, and the like in addition to general beverages and foods.

In addition to foods, the bifidobacteria of the present invention are also applicable in the fields of cosmetics such as a skin lotion, the field of pharmaceuticals such as an intestinal regulator, the field of daily necessities such as toothpaste, and the field of animal feeds and plant fertilizers such as silage, feed for animals, and liquid plant fertilizers.

### EXAMPLES

Hereinafter, examples of the present invention will be shown, but the present invention is not limited to the following examples.

### <Test Example 1> Evaluation Test on IL-10 and IL-12 Production Inducing Activity

The ability to induce IL-10 and IL-12 production was evaluated for the *Bifidobacterium breve* strain N708 of the present invention, three *Bifidobacterium breve* comparative strains (Bbr1, Bbr2, and Bbr3) owned by Nissin Foods Holdings Co., Ltd., and the comparative type strain *Bifidobacterium breve* JCM1192.

The ability to induce IL-10 and IL-12 production was evaluated according to the following procedure. The bifidobacteria of the present invention, the type strain, and the comparative strains (Bbr1, Bbr2, Bbr3) were each cultured in the GAM medium (GAM Broth "Nissui") shown in Table 3 at 37°C for 24 hours, and the grown cells were centrifuged and collected. The separated cells were washed with sterile water three times, sterilized by heat, and then frozen. Then, the cells were freeze-dried using a freeze dryer to obtain a dried killed bacterial cell powder.

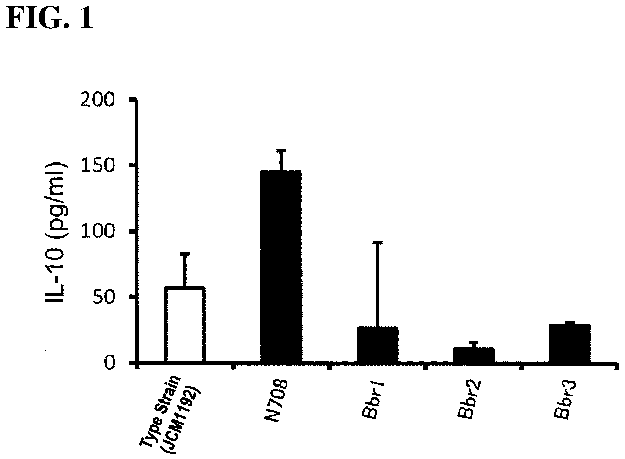

Human peripheral blood CD14+ monocytes were induced to differentiate into dendritic cells by culturing them in RPMI-1640 medium containing 20 ng/mL of GM-CS (Granulocyte Macrophage colony-stimulating Factor), 20 ng/mL of IL-4 and 10% FBS for 6 days under the conditions of 37°C and 5% CO₂. After the cells were collected, the cell count was adjusted, and 1 x 10⁵ cells/well were dispensed into a 96-well culture plate. The dried killed bacterial cell powder obtained above was added thereto to be 100 ng/mL, and then the mixture was cultured for 6 hours under the conditions of 37°C and 5% CO₂. Furthermore, an IL-12p70 inducer (0.1 µL/mL Lioppollysaccuaride, 20 ng/mL IFN-γ and 50 ng/mL TNF-α) was added and the mixture was cultured for 18 hours. After the culture, the culture supernatant was collected, and IL-10 in the culture supernatant was measured by the IL-10 Human Uncoated ELISA Kit (Invitrogen), and IL-12 was measured by Human IL-12p70 Uncoated ELISA (Invitrogen). The results are shown in Figures 1 to 3.

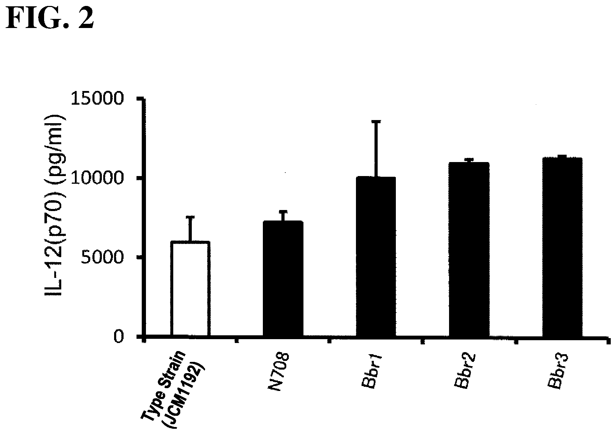

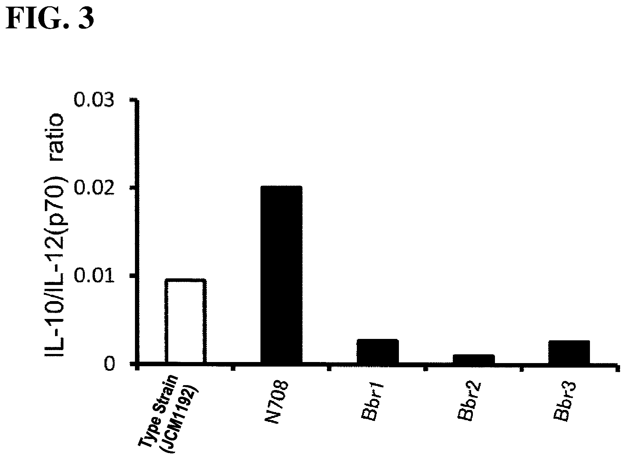

As is clear from Figure 1, the amount of IL-10 production of the *Bifidobacterium breve* strain N708 of the present invention was 145 pg/mL, which confirmed that the bifidobacteria of the present invention have a higher ability to induce IL-10 production than that of the type strain and the comparative strains (Bbr1, Bbr2, Bbr3). From Figure 2, it was also confirmed that the *Bifidobacterium breve* strain N708 does not induce IL-12 production compared to the other strains. Furthermore, as shown in Figure 3, it was possible to confirm that the ratio of the amount of IL-10 production/amount of IL-12 production of the *Bifidobacterium breve* strain N708 was significantly higher than those of the other strains.

### <Test Example 2> Skim Milk Medium Growth Test

A skim milk medium growth test was performed on the *Bifidobacterium breve* strain N708 of the present invention and the type strain *Bifidobacterium breve* JCM1192.

The bacterial cells were inoculated into a 10% SM (skim milk) + 0.5% yeast extract medium, which was cultured at 37°C for 24 hours to evaluate their growth in the skim milk medium by the viable cell count. The results are shown in Table 4.

**[Table 4]**

| Strain Name | Cell Count (x 10⁷ cfu/ml) |
|---|---|
| Bifidobacterium breve type Strain (JCM1192) | 4.8 |
| Bifidobacterium breve N708 | 92.1 |

The bacterial cell count of the *Bifidobacterium breve* strain N708 of the present invention was 9.21 x 10⁸ cfu/mL, which is a level at which there is no problem in producing fermented milk and lactic acid bacteria beverages.

## Claims

1. Bifidobacteria belonging to the genus Bifidobacterium, wherein the bifidobacteria is Bifidobacterium breve strain N708 (deposited at the Patent Microorganisms Depositary Center, National Institute of Technology and Evaluation with accession number NITE BP-02958 on May 29, 2019).

2. A food or drink product, comprising the bifidobacteria according to claim 1.

## Patentansprüche

1. Bifidobakterien, die zum Genus Bifidobacterium gehören, wobei die Bifidobakterien Bifidobacterium-breve-Stamm N708 (hinterlegt beim Patent Microorganisms Depositary Center, National Institute of Technology and Evaluation am 29. Mai 2019 unter der Zugriffsnummer NITE BP-02958) sind.

2. Nahrungsmittel- oder Getränkeprodukt, umfassend Bifidobakterien nach Anspruch 1.

## Revendications

1. Bifidobactéries appartenant au genre Bifidobacterium, dans lesquelles les bifidobactéries sont de la souche Bifidobacterium breve N708 (déposée au Patent Microorganisms Depositary Center, National Institute of Technology and Evaluation sous le numéro d'accession NITE BP-02958 le 29 mai 2019).

2. Aliment ou boisson, comprenant les bifidobactéries selon la revendication 1.
